# EUROPEAN PATENT APPLICATION

(11) **EP 4 806 488 A1**
(43) Date of publication of application: **16.09.2026**
(21) Application number: 24911128.7
(22) Date of filing: 24.12.2024
(51) Int. Cl.: A61M 21/00

(54) **FRAGRANCE RELEASE METHOD AND APPARATUS, FRAGRANCE RELEASE SYSTEM, AND STORAGE MEDIUM**

(30) Priority: 25.12.2023 CN 202311804803
(71) Applicant: Shanghai United Imaging Healthcare Co., Ltd., Shanghai 201807 (CN)
(72) Inventor: YIN, Shaoting, Shanghai 201807 (CN); TIAN, Zhengyang, Shanghai 201807 (CN)
(74) Representative: Murgitroyd & Company
(86) International application number: PCT/CN2024/141919
(87) International publication number: WO 2025/140203

(57) **Abstract**

The present application relates to a fragrance release method and apparatus, a fragrance release system, and a storage medium. The fragrance release method comprises: obtaining multi-modal data of a target object in a target medical space of a medical device, wherein the multi-modal data comprises at least one of physiological information, facial expression information, and vocal information; processing the multi-modal data using an emotion recognition model to obtain an emotion recognition result of the target object; and determining, based on examination information and the emotion recognition result of the target object, a target fragrance release mode for releasing fragrance for the target object.

## Description

### CROSS-REFERENCE TO RELATED APPLICATIONS

The present application claims priority to Chinese patent application No. 2023118048038, filed on December 25, 2023, and entitled "FRAGRANCE RELEASE METHOD AND APPARATUS, FRAGRANCE RELEASE SYSTEM, AND STORAGE MEDIUM", the entire content of which is incorporated herein by reference.

### TECHNICAL FIELD

The present disclosure relates to the field of medical equipment technologies, and in particular, to a fragrance release method and apparatus, a fragrance release system, a storage medium and a computer program product.

### BACKGROUND

With the development of medical equipment technology, treatment or diagnosis devices such as magnetic resonance and CT (computed tomography) equipment have emerged with large volumes. A subject often needs to enter a treatment space or a diagnosis area, such as the bore of a large medical imaging device, for a long-time scan. Many subjects find it difficult to stay calm in the treatment space or diagnosis area due to tension and anxiety, which may lead to movement artifacts affecting scan imaging at the least, intense emotions such as dizziness at worst, and even trigger claustrophobia.

However, in the present treatment/diagnosis process, there is a problem of insufficient breathing guidance.

### SUMMARY

Accordingly, a fragrance release method and apparatus, a fragrance release system, a storage medium and a computer program product are provided in the present disclosure.

In a first aspect, a fragrance release method is provided in the present disclosure. The method includes:
obtaining multi-modal data of a target object in a target medical space of a medical device, where the multi-modal data include at least one of physiological information, facial expression information, or vocal information;
processing the multi-modal data by using an emotion recognition model to obtain an emotion recognition result of the target object; and
determining a target fragrance release mode for releasing fragrance to the target object based on examination information of the target object and the emotion recognition result.

In one of the embodiments, determining the target fragrance release mode for releasing fragrance to the target object based on the examination information of the target object and the emotion recognition result includes:
obtaining the examination information of the target object, and determining a predetermined interaction mode by performing a matching operation based on the examination information; and
determining the target fragrance release mode based on the emotion recognition result and the predetermined interaction mode.

In one of the embodiments, the examination information includes treatment or diagnosis information of the medical device for the target object. The target fragrance release mode includes a target fragrance release time, a target fragrance release intensity, and a target fragrance type. The method further includes:
adjusting at least one of the target fragrance release time or the target fragrance release intensity based on the treatment or diagnosis information; and
determining the target fragrance type based on the emotion recognition result.

In one of the embodiments, obtaining the examination information of the target object, and determining the predetermined interaction mode by performing a matching operation based on the examination information includes:
determining whether to release fragrance to the target object based on obtained examination information; and
determining, in response to determining to release fragrance to the target object, the predetermined interaction mode by performing a matching operation based on the examination information.

In one of the embodiments, the method further includes:
obtaining and processing the multi-modal data in the target fragrance release mode to obtain feedback information of the target object for the target fragrance release mode; and
dynamically adjusting the target fragrance release mode based on the feedback information.

In one of the embodiments, the method further includes:
obtaining feature information obtained by processing the multi-modal data by the emotion recognition model;
performing enhanced training on the emotion recognition model based on the feature information to obtain an enhanced emotion recognition model and updated feature information; and
calibrating the enhanced emotion recognition model by using the updated feature information to obtain an updated emotion recognition model.

In one of the embodiments, the emotion recognition model includes a facial emotion model. The emotion recognition result includes a facial emotion intensity.

In one of the embodiments, the method further including:
processing the facial expression information by using a convolutional layer of the facial emotion model to obtain facial emotion features; and
processing the facial emotion features by using a pooling layer of the facial emotion model to obtain the facial emotion intensity.

In a second aspect, a fragrance release apparatus is provided in the present disclosure. The apparatus includes:
an information acquisition module configured to acquire multi-modal data of a target object in a target medical space of a medical device, where the multi-modal data include at least one of physiological information, facial expression information, or vocal information;
an emotion recognition module configured to process the multi-modal data by using an emotion recognition model to obtain an emotion recognition result of the target object; and
a mode determination module configured to determine a target fragrance release mode for releasing fragrance to the target object based on examination information of the target object and the emotion recognition result.

In a third aspect, a fragrance release system is provided in the present disclosure. The fragrance release system includes a processing unit configured to implement the steps of the above-mentioned method. The fragrance release system further includes an information acquisition assembly and a fragrance release apparatus. The information acquisition assembly includes an image acquisition module, a sound acquisition module and an information entry module all connected to the processing unit. The image acquisition module is configured to acquire the facial expression information. The sound acquisition module is configured to acquire the vocal information. The information entry module is configured to receive the physiological information. The fragrance release apparatus includes a fragrance generating module and a fragrance dispersing module both connected to the processing unit.

In a fourth aspect, a fragrance release control device is provided in the present disclosure. The fragrance release control device is configured to be connected to an image acquisition module, a sound acquisition module and an information entry module in an information acquisition assembly respectively. The image acquisition module is configured to acquire the facial expression information, the sound acquisition module is configured to acquire the vocal information, and the information entry module is configured to receive the physiological information. The fragrance release control device is further configured to be connected to a fragrance generating module and a fragrance dispersing module in a fragrance release apparatus respectively. The fragrance release control device includes a memory and a processor, the memory storing a computer program. The processor, when executing the computer program, implements the steps of the above-mentioned method.

In a fifth aspect, a medical device is provided in the present disclosure. The medical device includes a treatment or diagnosis device, and a supporting device for carrying a target object. The treatment or diagnosis device is configured to perform medical treatment or diagnosis on the target object. The medical device includes a memory and a processor, the memory storing a computer program. The processor, when executing the computer program, implements the steps of the above-mentioned method.

In a sixth aspect, a computer-readable storage medium including a computer program stored thereon is provided in the present disclosure. The computer program, when executed by a processor, causes the processor to implement the steps of the above-mentioned method.

In a seventh aspect, a computer program product including a computer program is provided in the present disclosure. The computer program, when executed by a processor, causes the processor to implement the steps of the above-mentioned method.

Details of one or more embodiments of the present disclosure are set forth in the accompanying drawings and the description below. Other features, objects, and advantages of the present disclosure will become apparent from the description, accompanying drawings, and claims.

### BRIEF DESCRIPTION OF THE DRAWINGS

In order to explain the technical solutions in the embodiments of the present disclosure or in the conventional technology more clearly, the accompanying drawings that need to be used in the description of the embodiments or the conventional technology will be briefly introduced below. Apparently, the accompanying drawings described below are only some embodiments of the present disclosure, and for those of ordinary skill in the art, other accompanying drawings can be obtained according to the disclosed accompanying drawings without creative efforts.
FIG. 1 is a flow diagram illustrating a fragrance release method in an embodiment of the present disclosure;
FIG. 2 is a flow diagram illustrating a fragrance release step in an embodiment of the present disclosure;
FIG. 3 is a flow diagram illustrating a fragrance release method in another embodiment of the present disclosure;
FIG. 4 is a flow diagram illustrating a fragrance release method in another embodiment of the present disclosure;
FIG. 5 is a flow diagram illustrating a fragrance release method in still another embodiment of the present disclosure;
FIG. 6 is a flow diagram illustrating a fragrance release step in another embodiment of the present disclosure;
FIG. 7 is a block diagram illustrating a configuration of a fragrance release apparatus in an embodiment of the present disclosure;
FIG. 8 is a block diagram illustrating a configuration of a fragrance release system in an embodiment of the present disclosure;
FIG. 9 is a schematic diagram illustrating an internal configuration of a fragrance release control device in an embodiment of the present disclosure; and
FIG. 10 is a schematic diagram illustrating an internal configuration of a fragrance release control device in another embodiment of the present disclosure.

### DETAILED DESCRIPTION OF THE EMBODIMENTS

In order to make the objectives, technical solutions and advantages of the present disclosure clearer, the present disclosure will be described in further detail below with reference to the accompanying drawings and embodiments. It should be understood that the specific embodiments described herein are only used to explain the present disclosure, and are not intended to limit the present disclosure.

At present, in a space for treatment or diagnosis provided by a medical device for a subject, such as a scan room of an imaging device, the care for the subject is mainly focused on the audiovisual aspect. While at the olfactory level of the five senses, a one-way output fragrance system is usually adopted. The fragrance system occupies the olfactory sense of the subject for a long time by releasing fragrance, and there is no interactive change between the fragrance system and the subject. This solution still has the problem of insufficient breathing guidance for the subject.

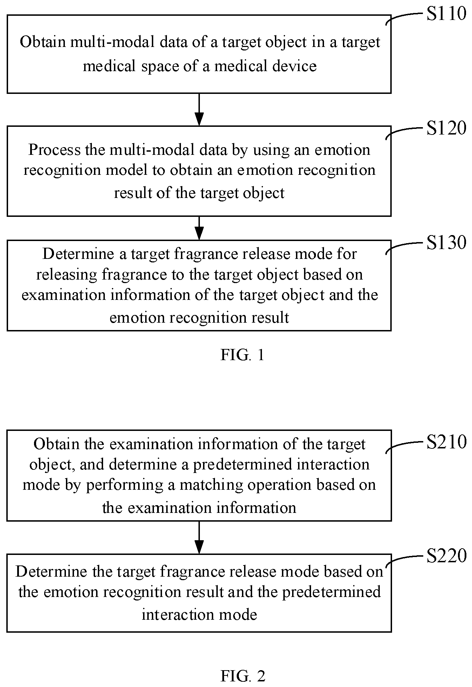

In an embodiment, as shown in FIG. 1, a fragrance release method is provided, the method includes steps S110 to S130.

In step S110, multi-modal data of a target object in a target medical space of a medical device are obtained. The multi-modal data include at least one of physiological information, facial expression information, or vocal information.

Specifically, the fragrance release method can be applied to a processing unit of a fragrance release system, or to a medical device. The medical device may include a treatment or diagnosis device, and a supporting device for carrying a target object, and the treatment or diagnosis device is configured to perform medical treatment or diagnosis on the target object. The treatment or diagnosis device may include medical imaging devices such as an X-ray scanning device, a computed tomography device, a magnetic resonance imaging device, a positron emission computed tomography device, and a gamma camera. The treatment or diagnosis device may further include a radiation therapy device, an ultrasonic diagnosis device, a dialyzer, a laser therapy device, a blood glucose monitor, an electrocardiograph and other devices. The supporting device can be used to support the target object and also support medical devices. For example, the supporting device may include a scanning table, a hospital bed, a wheelchair, a bracket and the like, so as to ensure that the target object can maintain an appropriate posture or a safe position when receiving treatment or diagnosis, and the treatment space or diagnosis area can accommodate the supporting device. The target medical space may include a treatment space or a diagnosis area of the medical device. On a condition that a subject undergoes medical treatment or diagnosis with the medical device, the subject enters the treatment space or the diagnosis area. For example, the subject lies on a scanning table inside the bore of a large medical imaging device, the subject can be determined as the target object, and then the multi-modal data of the target object can be collected. The multi-modal data include at least one of physiological information, facial expression information, or vocal information. The physiological information of the target object may include electrocardiogram data, respiratory data, blood pressure data and the like of the target object. The facial expression information of the target object may include expressions such as smiling, frowning, blinking of the target object, and the activity of facial muscles. The vocal information of the target object may include speaking speed, tone, voice emotion and other information of the target object.

In some examples, physiological information of the target object may be collected, and the physiological information is processed by using an emotion recognition model to obtain an emotion recognition result of the target object. The emotion recognition model may include a physiological emotion model. By using the physiological emotion model, at least one of electrocardiogram data, respiratory data, blood pressure data, or other data of the target object may be processed to obtain the emotion recognition result of the target object.

In some examples, facial expression information of the target object may be collected, and the facial expression information is processed by using an emotion recognition model to obtain an emotion recognition result of the target object. The emotion recognition model may include a facial emotion model. By using the facial emotion model, at least one of expressions such as smiling, frowning, blinking of the target object and the activity of facial muscles may be processed to obtain the emotion recognition result of the target object. The emotion recognition result may include a facial emotion intensity.

In some examples, vocal information of the target object may be collected, and the vocal information is processed by using an emotion recognition model to obtain an emotion recognition result of the target object. The emotion recognition model may include a voice emotion model. By using the voice emotion model, at least one of speaking speed, tone, voice emotion, or other information of the target object may be processed to obtain the emotion recognition result of the target object.

In some examples, a plurality of types of information among physiological information, facial expression information, and vocal information of the target object may be collected, and corresponding emotion recognition models are respectively used to process different types of information, or a trained emotion recognition model capable of comprehensively processing a plurality of types of information is used to obtain the emotion recognition result of the target object.

By obtaining the above multi-modal data, the state of the target object in the treatment space or diagnosis area can be obtained in real time, and then the released fragrance can be adjusted based on the state of the target object, so as to improve the interactivity with the target object in the fragrance release process and enhance the effect of breathing guidance.

In some examples, the physiological information, facial expression information, and vocal information of the target object may be collected based on an information acquisition assembly of the fragrance release system. The information acquisition assembly may include an image acquisition module, a sound acquisition module and an information entry module all connected to the processing unit. The image acquisition module can collect facial expression information, and the image acquisition module can be implemented by using at least one of a space camera, a bore camera, or a millimeter wave radar. For example, the bore camera can collect facial expression information, and then the processing unit can perform expression analysis and facial micro-movement recognition on the target object, including but not limited to the tension degree, fatigue degree, respiratory rate, whether to fall asleep and the like of the target object. Based on the image information collected by the space camera, the processing unit can collect and identify the position of the target object, and take the position of the target object as a part for turning on or controlling the fragrance release system. The sound acquisition module can collect vocal information of the target object, and the sound acquisition module may include a microphone. The microphone can collect vocal information of the target object, such as vocal information subconsciously provided from the target object like "It stinks, what's the smell". The information entry module can be configured to connect to corresponding data interfaces. For example, the information entry module can connect various sensors worn by the target object during scanning, and collect physiological information including various information such as respiration, electrocardiogram, blood pressure through the sensors.

In step S120, the multi-modal data are processed by using an emotion recognition model to obtain an emotion recognition result of the target object.

Specifically, the obtained multi-modal data can be processed by using an emotion recognition model. The emotion recognition model may be a convolutional neural network model for identifying the comprehensive emotional state of the target object. The obtained emotion recognition result of the target object may include various emotional states such as happiness, sadness, anger, anxiety and the like. The emotion recognition result of the target object may further include an emotion intensity, which may be a quantitative embodiment of the corresponding emotional state. Through the above method, the comprehensive emotional state of the target object including the present potential emotion can be identified, and then the release of fragrance can be controlled based on the comprehensive emotional state of the target object.

In some examples, the obtained multi-modal data can be stored in a corresponding database. For example, physiological information is stored in a physiological information database, facial expression information is stored in a facial expression information database, and vocal information is stored in a vocal information database, and the various databases can be linked with each other. The emotion recognition model can combine a plurality of databases to comprehensively analyze the realtime emotional information of the target object from visual and auditory perspectives. Physiological information, facial expression information, and vocal information can serve as inputs of the emotion recognition model, and the emotion recognition result of the target object can serve as an output of the emotion recognition model. The emotion recognition model can also be enhanced by artificial intelligence (AI) combined with big data to obtain the emotional state and emotion intensity of the target object. For example, a corresponding index can be specified to enhance the emotional state and emotion intensity, and a certain emotional state is achieved after reaching the index, and the output emotion intensity is obtained by enhancement within a corresponding range. For example, the emotion recognition result of the target object may be anxiety, 70%. The emotion intensity may include a facial emotion intensity.

In step S130, a target fragrance release mode for releasing fragrance to the target object can be determined based on examination information of the target object and the emotion recognition result.

Specifically, the target fragrance release mode for releasing fragrance to the target object can be determined based on the examination information of the target object and the emotion recognition result. The examination information of the target object may include information such as age, gender, medical record information, scanning content, scanning duration and whether there is a fragrance allergy history of the target object. Fragrance release modes preset for different target objects and different protocols can be set to interact with the target object to realize breathing guidance. For example, based on the olfactory differences between the elderly and young people, different fragrance release modes can be set, and then a personalized target fragrance release mode can be determined by performing a matching operation for the present target object, which improves the effectiveness of breathing guidance.

In the embodiment of the present disclosure, multi-modal data of a target object in a target medical space of a medical device are collected. The multi-modal data include at least one of physiological information, facial expression information, or vocal information, so as to obtain the state of the target object in the treatment space or diagnosis area of the medical device in real time. The multi-modal data are processed by using an emotion recognition model to obtain an emotion recognition result of the target object, so as to identify the comprehensive emotional state of the target object including the present potential emotion. A target fragrance release mode for releasing fragrance to the target object are determined based on the examination information of the target object and the emotion recognition result, to improve the interactivity with the target object in the fragrance release process, thus improving the effect of breathing guidance.

In one of the embodiments, as shown in FIG. 2, determining the target fragrance release mode for releasing fragrance to the target object based on the examination information of the target object and the emotion recognition result includes steps S210 to S220.

In step S210, the examination information of the target object is obtained, and a predetermined interaction mode is determined by performing a matching operation based on the examination information.

In step S220, the target fragrance release mode is determined based on the emotion recognition result and the predetermined interaction mode.

Specifically, the examination information of the target object may include information such as age, gender, medical record information, scanning content, scanning duration and whether there is a fragrance allergy history of the target object. The predetermined interaction mode determined by performing a matching operation based on the examination information may be one of a variety of pre-stored olfactory interaction modes, and the variety of pre-stored olfactory interaction modes may be predetermined based on historical examination information of different historical objects to adapt to different target objects. Further, the target fragrance release mode corresponding to the emotion recognition result under the predetermined interaction mode can be determined based on the emotion recognition result and the predetermined interaction mode, and then the processing unit can control the fragrance release apparatus to work in the target fragrance release mode.

In some examples, based on obtained examination information of the target object, it can also be first determined whether to release fragrance to the target object, and the predetermined interaction mode is determined by performing a matching operation based on the examination information when it is determined to use the fragrance release system for the target object. For example, based on obtained examination information of the target object, if it is determined that the target object is one of the following, the fragrance release system is not used for the target object: people with allergic constitution, asthmatic patients, people with skin sensitivity, pregnant women, infants and young children, patients with specific diseases and the like.

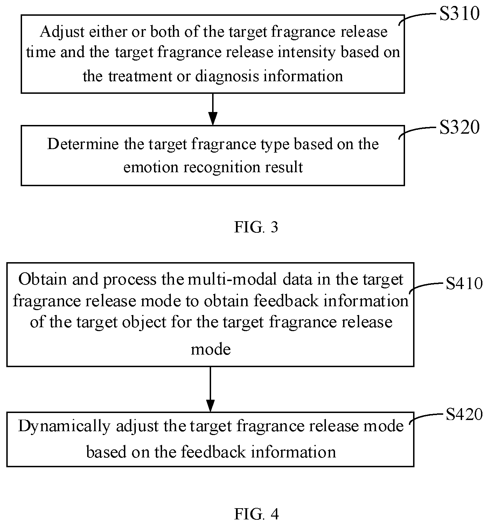

In one of the embodiments, the examination information includes treatment or diagnosis information of the medical device for the target object. The target fragrance release mode includes a target fragrance release time, a target fragrance release intensity, and a target fragrance type. As shown in FIG. 3, the method further includes steps S310 to S320.

In step S310, either or both of the target fragrance release time and the target fragrance release intensity is adjusted based on the treatment or diagnosis information.

In step S320, the target fragrance type is determined based on the emotion recognition result.

Specifically, the treatment or diagnosis information for the target object may include mode information and parameter information that need to be set for the treatment or diagnosis device for the target object. On a condition that the treatment or diagnosis device is a medical imaging device, the treatment or diagnosis information may include scanning information such as a scanning mode and scanning parameters, for example, information such as scanning content and scanning duration. On a condition that the treatment or diagnosis device is a radiation therapy device, the treatment or diagnosis information may include radiation dose, treatment site, treatment target, treatment cycle, setting parameters of the radiation therapy device and the like. On a condition that the treatment or diagnosis device is an ultrasonic diagnosis device, the treatment or diagnosis information may include ultrasonic parameters, examination mode and the like. The processing unit can adjust either or both of the target fragrance release time and the target fragrance release intensity based on the treatment or diagnosis information. The target fragrance release time may be the time for the fragrance release apparatus of the fragrance release system to release fragrance, and the target fragrance release intensity may be the intensity for the fragrance release apparatus of the fragrance release system to release fragrance. The target fragrance type can be determined based on the emotion recognition result. For example, different fragrance types can be pre-matched with different emotional states and emotion intensities, so that the corresponding target fragrance type can be determined by performing a matching operation based on the obtained emotion recognition result. The target fragrance type may be a type of fragrance to be released by the fragrance release apparatus of the fragrance release system. Through the above method, the present emotional state and potential emotional trend of the target object can be analyzed, and the pre-designed fragrance type can be determined by performing a matching operation correspondingly, so as to improve the efficiency of breathing guidance.

In one of the embodiments, as shown in FIG. 4, the method further includes steps S410 to S420.

In step S410, the multi-modal data are obtained and processed in the target fragrance release mode to obtain feedback information of the target object for the target fragrance release mode.

In step S420, the target fragrance release mode is dynamically adjusted based on the feedback information.

Specifically, on a condition that the processing unit controls the fragrance release apparatus to be in the target fragrance release mode, the target object may make corresponding feedback for the target fragrance release mode. For example, in the case that the fragrance intensity released during breathing guidance is 40%, a target object about 50 years old may frown. The multi-modal data of the target object in this process can be continuously obtained, and the above information is processed to extract the feedback information of the target object for the target fragrance release mode. Then, the target fragrance release mode can be dynamically adjusted based on the feedback information, so as to improve the interactivity with the target object in the fragrance release process, thus improving the effectiveness of breathing guidance.

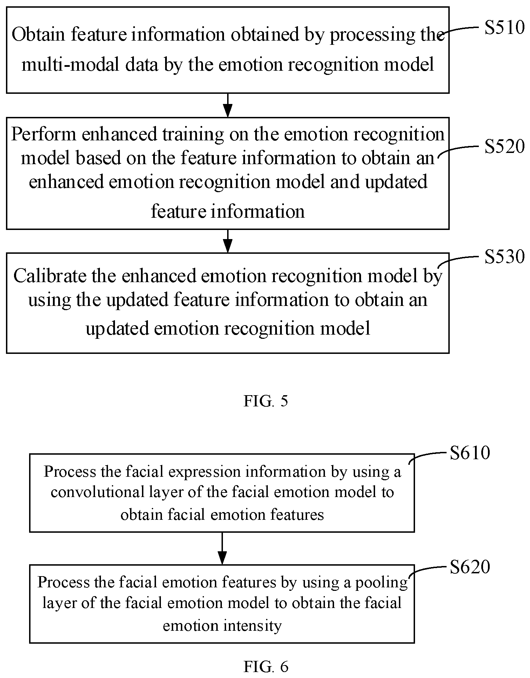

In one of the embodiments, as shown in FIG. 5, the method further includes steps S510 to S530.

In step S510, feature information obtained by processing the multi-modal data by the emotion recognition model are obtained.

In step S520, enhanced training is performed on the emotion recognition model based on the feature information to obtain an enhanced emotion recognition model and updated feature information.

In step S530, the enhanced emotion recognition model is calibrated by using the updated feature information to obtain an updated emotion recognition model.

Specifically, in the process of processing the multi-modal data by the emotion recognition model, the convolutional network can extract corresponding feature information, which can be configured to reflect the emotional features of the target object. The obtained feature information can be used for enhanced training of the emotion recognition model. For example, enhanced training can be performed in the actual use process of the emotion recognition model, or the emotion recognition model can be further trained after collecting various feature information and uploading it to a server. After performing enhanced training on the emotion recognition model, the emotion recognition model obtained by enhanced training can update the feature information, and the updated feature information is configured to calibrate the enhanced emotion recognition model. For example, the enhanced emotion recognition model that passes the calibration is taken as the updated emotion recognition model, so as to improve the generalization ability and recognition accuracy of the emotion recognition model. The updated emotion recognition model can provide personalized fragrance release schemes for different target objects. Through enhanced training, the feature information obtained in the use process of the emotion recognition model can be continuously used for training the emotion recognition model to improve the performance of the emotion recognition model.

In some embodiments, the emotion recognition model includes a facial emotion model. The emotion recognition result includes a facial emotion intensity.

Specifically, the facial expression information includes the expression of each emotion on the face, which can be described by the changes of various parts such as eyebrows, mouth corners, eyes and the like, and the intensity of facial emotion can be evaluated by processing the facial expression information, i.e., the facial emotion intensity is obtained.

In one of the embodiments, as shown in FIG. 6, the method further includes steps S610 to S620.

In step S610, the facial expression information is processed by using a convolutional layer of the facial emotion model to obtain facial emotion features.

In step S620, the facial emotion features are processed by using a pooling layer of the facial emotion model to obtain the facial emotion intensity.

Specifically, the facial emotion model may be a pre-trained convolutional neural network model. For example, it is trained by fixed samples, can be recorded in subsequent use, and can also be modified by combining physiological data of other modalities. The convolutional layer of the facial emotion model can be used to process facial expression information and extract facial emotion features to identify emotions such as anger, rage and the like. The pooling layer of the facial emotion model can further process the obtained facial emotion features to realize the measurement and output of the facial emotion intensity. The pooling layer may include standard pooling layers of different levels.

In some examples, the facial emotion features and facial emotion intensity of the target object can be identified by using a convolutional neural network model. Further, the processing unit can comprehensively analyze the emotional state and emotion intensity of the target object by combining vision, sound and the like to reflect the potential emotion of the target object other than facial expressions. For example, the target object is actually nervous and in pain but has been enduring. The processing unit can combine physiological information data such as respiration, for example, collect relevant physiological information data indicating that the respiration of the target object has been light and the heart rate is high through corresponding sensors. The processing unit determines the potential condition of the target object based on physiological information, facial expression information, and vocal information to obtain the emotion recognition result of the target object. Then, the processing unit can determine a corresponding target fragrance release mode. The processing unit controls the fragrance release apparatus of the fragrance release system to release fragrance in the target fragrance release mode. Thus, the target object is comforted from the olfactory perspective. The whole process is intelligently controlled without the intervention of a technician. In the above way, the fragrance release system can synchronously perceive the emotional state of the target object, and continuously adjust the target fragrance release mode based on the feedback of the target object. This improves the comfort of the target object during the scanning process. The system also conforms to the sensory characteristics of olfactory personalization, and enhance the effect of breathing guidance for the target object during the scanning process.

It should be understood that although the various steps in the flow charts involved in the above embodiments are shown in sequence according to the arrows, these steps are not necessarily executed in sequence according to the direction of the arrows. Unless explicitly stated herein, the execution of these steps has no strict order limit, and these steps can be executed in other orders. Moreover, at least some of the steps in the flow charts involved in the above embodiments may include a plurality of steps or a plurality of stages, which are not necessarily executed at the same time, but can be executed at different times, and the execution order of these steps or stages is not necessarily sequential, but can be executed alternately or alternately with at least some of the other steps or stages in the other steps.

Based on the same inventive concept, in the embodiments of the present disclosure, a fragrance release apparatus for implementing the above-mentioned fragrance release method is further provided. The implementation scheme for solving the problem provided by the apparatus is similar to the implementation scheme disclosed in the above-mentioned method, so the specific limitations in one or more embodiments of the fragrance release apparatus provided below can be referred to the limitations on the fragrance release method above, and will not be repeated here.

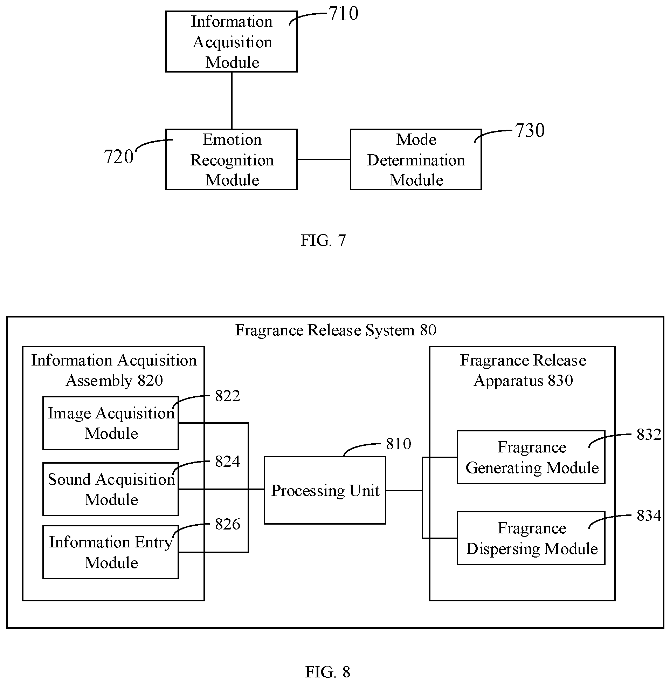

In an embodiment, as shown in FIG. 7, a fragrance release apparatus is provided. The apparatus includes an information acquisition module 710, an emotion recognition module 720, and a mode determination module 730.

The information acquisition module 710 is configured to acquire multi-modal data of a target object in a target medical space of a medical device. The multi-modal data include at least one of physiological information, facial expression information, or vocal information.

The emotion recognition module 720 is configured to process the multi-modal data by using an emotion recognition model to obtain an emotion recognition result of the target object.

The mode determination module 730 is configured to determine a target fragrance release mode for releasing fragrance to the target object based on examination information of the target object and the emotion recognition result.

In one of the embodiments, the mode determination module 730 is further configured to acquire the examination information of the target object, and determine a predetermined interaction mode by performing a matching operation based on the examination information, and determine the target fragrance release mode based on the emotion recognition result and the predetermined interaction mode.

In one of the embodiments, the examination information includes treatment or diagnosis information of the medical device for the target object. The target fragrance release mode includes a target fragrance release time, a target fragrance release intensity, and a target fragrance type. The apparatus further includes a fragrance adjustment module and a target fragrance type determination module.

The fragrance adjustment module is configured to adjust either or both of the target fragrance release time and the target fragrance release intensity based on the treatment or diagnosis information.

The target fragrance type determination module is configured to determine the target fragrance type based on the emotion recognition result.

In one of the embodiments, the mode determination module 730 is further configured to determine whether to release fragrance to the target object based on obtained examination information, and in response to determining to release fragrance to the target object, determine the predetermined interaction mode by performing a matching operation based on the examination information.

In one of the embodiments, the apparatus further includes a feedback information acquisition module and a dynamic adjustment module.

The feedback information acquisition module is configured to acquire and process the multi-modal data in the target fragrance release mode to obtain feedback information of the target object for the target fragrance release mode.

The dynamic adjustment module is configured to dynamically adjust the target fragrance release mode based on the feedback information.

In one of the embodiments, the apparatus further includes a feature information acquisition module, a feature information update module, and an emotion recognition model update module.

The feature information acquisition module is configured to acquire feature information obtained by processing the multi-modal data by the emotion recognition model.

The feature information update module is configured to perform enhanced training on the emotion recognition model based on the feature information to obtain an enhanced emotion recognition model and updated feature information.

The emotion recognition model update module is configured to calibrate the enhanced emotion recognition model by using the updated feature information to obtain an updated emotion recognition model.

In one of the embodiments, the emotion recognition model includes a facial emotion model. The emotion recognition result includes a facial emotion intensity.

In one of the embodiments, the apparatus further includes a facial emotion feature acquisition module and a facial emotion intensity acquisition module.

The facial emotion feature acquisition module is configured to process the facial expression information by using a convolutional layer of the facial emotion model to obtain facial emotion features.

The facial emotion intensity acquisition module is configured to process the facial emotion features by using a pooling layer of the facial emotion model to obtain the facial emotion intensity.

Each module in the above fragrance release apparatus can be implemented in whole or in part by software, hardware and a combination thereof. Each of the above modules can be embedded in or independent of a processor in the fragrance release control device in the form of hardware, or stored in a memory in the fragrance release control device in the form of software, so that the processor can call and execute the operations corresponding to each of the above modules.

In an embodiment, as shown in FIG. 8, a fragrance release system 80 is provided. The fragrance release system 80 includes a processing unit 810. The processing unit 810 is configured to implement the steps of the above-mentioned method. The fragrance release system 80 further includes an information acquisition assembly 820 and a fragrance release apparatus 830. The information acquisition assembly 820 includes an image acquisition module 822, a sound acquisition module 824 and an information entry module 826 all connected to the processing unit 810. The fragrance release apparatus 830 includes a fragrance generating module 832 and a fragrance dispersing module 834 both connected to the processing unit 810.

Specifically, the image acquisition module 822 can collect facial expression information, and the image acquisition module 822 can be implemented by using at least one of a space camera, a bore camera, or a millimeter wave radar. The sound acquisition module 824 can collect vocal information of the target object, and the sound acquisition module 824 may include a microphone, which can collect vocal information of the target object. The information entry module 826 can be configured to connect to corresponding data interfaces. For example, the information entry module 826 can be configured to connect to various sensors worn by the target object during scanning, and collect physiological information including various information such as respiration, electrocardiogram, blood pressure through the sensors. The fragrance release apparatus 830 can store a variety of fragrance agents. The fragrance release apparatus 830 includes a fragrance generating module 832 and a fragrance dispersing module 834. The fragrance generating module 832 may include either or both of an integrator and a dispenser. The integrator can store a variety of pre-prepared fragrance agents with fragrance types, and the dispenser can be filled with several fragrance base materials that can be mixed into different fragrances. The fragrance dispersing module 834 may include an air supply cylinder and an air exhaust cylinder, so as to realize rapid fragrance dispersion and exhaust under the control of the processing unit 810 in a treatment space or a diagnosis area, for example, inside the bore of a scanning device or other small spaces.

In an embodiment, a computer-readable storage medium is provided. The computer-readable storage medium including a computer program stored thereon. The computer program, when executed by a processor, causes the processor to implement the steps of the above-mentioned method.

In an embodiment, a computer program product is provided. The computer program product includes a computer program. The computer program, when executed by a processor, causes the processor to implement the steps of the above-mentioned method.

In an embodiment, a fragrance release control device is provided. The fragrance release control device is configured to be connected to an image acquisition module, a sound acquisition module and an information entry module in an information acquisition assembly respectively. The image acquisition module is configured to acquire facial expression information. The sound acquisition module is configured to acquire vocal information. The information entry module is configured to receive physiological information. The fragrance release control device is further configured to be connected to a fragrance generating module and a fragrance dispersing module in a fragrance release apparatus respectively. The fragrance release control device includes a memory and a processor. The memory stores a computer program. The processor, when executing the computer program, implements the steps of the above-mentioned method.

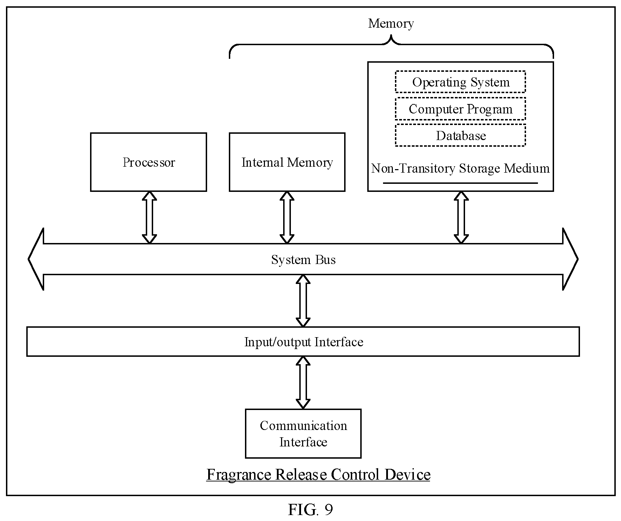

In an embodiment, a fragrance release control device is provided, which may be a server, and its schematic diagram illustrating an internal configuration may be as shown in FIG. 9. The fragrance release control device includes a processor, a memory, an input/output (I/O) interface and a communication interface. The processor, the memory and the input/output interface are connected through a system bus, and the communication interface is connected to the system bus through the input/output interface. The processor of the fragrance release control device is configured to provide computing and control capabilities. The memory of the fragrance release control device includes a non-transitory storage medium and an internal memory. The non-transitory storage medium stores an operating system, a computer program and a database. The internal memory provides an environment for the operation of the operating system and the computer program in the non-transitory storage medium. The database of the fragrance release control device is configured to store fragrance release data. The input/output interface of the fragrance release control device is configured to exchange information between the processor and external devices. The communication interface of the fragrance release control device is configured to communicate with an external terminal through a network connection. The computer program, when executed by a processor, causes the processor to implement steps of a fragrance release method.

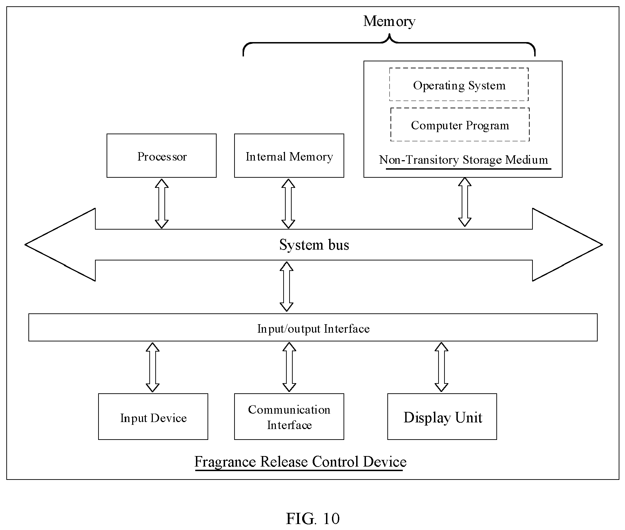

In an embodiment, a fragrance release control device is provided, which may be a terminal, and its schematic diagram illustrating an internal configuration may be as shown in FIG. 10. The fragrance release control device includes a processor, a memory, an input/output interface, a communication interface, a display unit and an input device. The processor, the memory and the input/output interface are connected through a system bus, and the communication interface, the display unit and the input device are connected to the system bus through the input/output interface. The processor of the fragrance release control device is configured to provide computing and control capabilities. The memory of the fragrance release control device includes a non-transitory storage medium and an internal memory. The non-transitory storage medium stores an operating system and a computer program. The internal memory provides an environment for the operation of the operating system and the computer program in the non-transitory storage medium. The input/output interface of the fragrance release control device is configured to exchange information between the processor and external devices. The communication interface of the fragrance release control device is configured to communicate with an external terminal in a wired or wireless manner, and the wireless manner can be realized through Wi-Fi, a mobile cellular network, near field communication (NFC) or other technologies. The computer program, when executed by the processor, causes the processor to implement a fragrance release method. The display unit of the fragrance release control device is configured to form a visually visible picture, and may be a display screen, a projection device or a virtual reality imaging device. The display screen may be a liquid crystal display screen or an electronic ink display screen, and the input device of the fragrance release control device may be a touch layer covered on the display screen, or keys, a trackball or a touch pad arranged on the housing of the fragrance release control device, or an external keyboard, touch pad or mouse and the like.

Those skilled in the art can understand that the structures shown in FIGS. 9 and 10 are only block diagrams of partial structures related to the solution of the present disclosure, and do not constitute a limitation on the fragrance release control device to which the solution of the present disclosure is applied. The specific fragrance release control device may include more or fewer components than those shown in the figures, or combine some components, or have different component arrangements.

It should be noted that the physiological information, facial expression information, vocal information, examination information, treatment or diagnosis information, feedback information and the like involved in the present disclosure are all information and data authorized by the user or fully authorized by all parties, and the collection, use and processing of relevant data need to comply with the relevant laws, regulations and standards of relevant countries and regions.

Those of ordinary skill in the art can understand that all or part of the processes in the methods of the above embodiments can be completed by instructing the relevant hardware through a computer program, and the computer program can be stored in a non-transitory computer-readable storage medium. When the computer program is executed, it may include the processes of the above method embodiments. Any reference to a memory, a database or other media involved in the various embodiments provided in the present disclosure may include either or both of a non-transitory memory and a volatile memory. The non-transitory memory may include a read-only memory (ROM), a magnetic tape, a floppy disk, a flash memory, an optical memory, a high-density embedded non-transitory memory, a resistive random access memory (ReRAM), a magnetoresistive random access memory (MRAM), a ferroelectric random access memory (FRAM), a phase change memory (PCM), a graphene memory and the like. The volatile memory may include a random access memory (RAM) or an external high-speed cache memory and the like. For illustration and not limitation, the RAM may be in various forms, such as a static random access memory (SRAM) or a dynamic random access memory (DRAM) and the like. The database involved in the various embodiments provided in the present disclosure may include either or both of a relational database and a non-relational database. The non-relational database may include a distributed database based on a blockchain and the like, without limitation. The processor involved in the various embodiments provided in the present disclosure may be a general-purpose processor, a central processing unit, a graphics processor, a digital signal processor, a programmable logic device, a data processing logic device based on quantum computing and the like, without limitation.

The technical features of the above embodiments can be combined arbitrarily. For the sake of concise description, all possible combinations of the technical features in the above embodiments are not described. However, as long as there is no contradiction in the combination of these technical features, all of them should be regarded as the scope disclosed in this specification.

The above embodiments only express several implementation modes of the present disclosure, and their descriptions are relatively specific and detailed, but they should not be construed as a limitation on the patent scope of the present disclosure. It should be pointed out that for those of ordinary skill in the art, without departing from the concept of the present disclosure, several modifications and improvements can be made, which all fall within the protection scope of the present disclosure. Therefore, the protection scope of the present disclosure shall be subject to the appended claims.

## Claims

1. A fragrance release method, comprising:
obtaining multi-modal data of a target object in a target medical space of a medical device, wherein the multi-modal data comprises at least one of physiological information, facial expression information, or vocal information;
processing the multi-modal data by using an emotion recognition model to obtain an emotion recognition result of the target object; and
determining a target fragrance release mode for releasing fragrance to the target object based on examination information of the target object and the emotion recognition result.

2. The fragrance release method according to claim 1, wherein determining the target fragrance release mode for releasing fragrance to the target object based on the examination information of the target object and the emotion recognition result comprises:
obtaining the examination information of the target object, and determining a predetermined interaction mode by performing a matching operation based on the examination information; and
determining the target fragrance release mode based on the emotion recognition result and the predetermined interaction mode.

3. The fragrance release method according to any one of claims 1 to 2, wherein the examination information comprises treatment or diagnosis information of the medical device for the target object; the target fragrance release mode comprises a target fragrance release time, a target fragrance release intensity, and a target fragrance type, and the method further comprises:
adjusting either or both of the target fragrance release time and the target fragrance release intensity based on the treatment or diagnosis information; and
determining the target fragrance type based on the emotion recognition result.

4. The fragrance release method according to claim 2, wherein obtaining the examination information of the target object, and determining the predetermined interaction mode by performing a matching operation based on the examination information comprises:
determining whether to release fragrance to the target object based on obtained examination information; and
determining, in response to determining to release fragrance to the target object, the predetermined interaction mode by performing a matching operation based on the examination information.

5. The fragrance release method according to claim 2, wherein the method further comprises:
obtaining and processing the multi-modal data in the target fragrance release mode to obtain feedback information of the target object for the target fragrance release mode; and
dynamically adjusting the target fragrance release mode based on the feedback information.

6. The fragrance release method according to claim 5, wherein the method further comprises:
obtaining feature information obtained by processing the multi-modal data by the emotion recognition model;
performing enhanced training on the emotion recognition model based on the feature information to obtain an enhanced emotion recognition model and updated feature information; and
calibrating the enhanced emotion recognition model by using the updated feature information to obtain an updated emotion recognition model.

7. The fragrance release method according to any one of claims 1 to 6, wherein the emotion recognition model comprises a facial emotion model; and the emotion recognition result comprises a facial emotion intensity.

8. The fragrance release method according to claim 7, wherein the method further comprises:
processing the facial expression information by using a convolutional layer of the facial emotion model to obtain facial emotion features; and
processing the facial emotion features by using a pooling layer of the facial emotion model to obtain the facial emotion intensity.

9. A fragrance release apparatus, comprising:
an information acquisition module configured to acquire multi-modal data of a target object in a target medical space of a medical device, wherein the multi-modal data comprises at least one of physiological information, facial expression information, or vocal information;
an emotion recognition module configured to process the multi-modal data by using an emotion recognition model to obtain an emotion recognition result of the target object; and
a mode determination module configured to determine a target fragrance release mode for releasing fragrance to the target object based on examination information of the target object and the emotion recognition result.

10. The fragrance release apparatus according to claim 9, wherein the mode determination module is further configured to acquire the examination information of the target object, and determine a predetermined interaction mode by performing a matching operation based on the examination information; and determine the target fragrance release mode based on the emotion recognition result and the predetermined interaction mode.

11. A fragrance release system, the fragrance release system comprising a processing unit configured to implement the steps of the fragrance release method according to any one of claims 1 to 8, and the fragrance release system further comprising an information acquisition assembly and a fragrance release apparatus, wherein
the information acquisition assembly comprises an image acquisition module, a sound acquisition module and an information entry module all connected to the processing unit, the image acquisition module being configured to acquire the facial expression information, the sound acquisition module being configured to acquire the vocal information, and the information entry module being configured to receive the physiological information; and
the fragrance release apparatus comprises a fragrance generating module and a fragrance dispersing module both connected to the processing unit.

12. The fragrance release system according to claim 11, wherein the image acquisition module comprises at least one of a space camera, a bore camera, or a millimeter wave radar.

13. The fragrance release system according to claim 11 or 12, wherein the sound acquisition module comprises a microphone.

14. The fragrance release system according to any one of claims 11 to 13, wherein the information entry module is configured to connect to a data interface of a sensor for obtaining the physiological information.

15. A fragrance release control device, the fragrance release control device being configured to be connected to an image acquisition module, a sound acquisition module and an information entry module in an information acquisition assembly respectively, wherein the image acquisition module is configured to acquire the facial expression information, the sound acquisition module is configured to acquire the vocal information, and the information entry module is configured to receive the physiological information; the fragrance release control device is further configured to be connected to a fragrance generating module and a fragrance dispersing module in a fragrance release apparatus respectively; and the fragrance release control device comprises a memory and a processor, the memory storing a computer program, and the processor, when executing the computer program, implementing the steps of the fragrance release method according to any one of claims 1 to 8.

16. A medical device, comprising a treatment or diagnosis device, and a supporting device for carrying a target object, the treatment or diagnosis device being configured to perform medical treatment or diagnosis on the target object, wherein the medical device comprises a memory and a processor, the memory storing a computer program, and the processor, when executing the computer program, implementing the steps of the method according to any one of claims 1 to 8.

17. The medical device according to claim 16, the medical device further comprising an information acquisition assembly and a fragrance release apparatus, wherein the information acquisition module comprises an image acquisition module, a sound acquisition module and an information entry module all connected to the processing unit, the image acquisition module being configured to acquire the facial expression information, the sound acquisition module being configured to acquire the vocal information, and the information entry module being configured to receive the physiological information; and the fragrance release apparatus comprises a fragrance generating module and a fragrance dispersing module both connected to the processing unit.

18. The medical device according to claim 16 or 17, wherein the treatment or diagnosis device comprises at least one of a radiation therapy device, an X-ray scanning device, a computed tomography device, a magnetic resonance imaging device, a positron emission computed tomography device, or an ultrasonic diagnosis device.

19. The medical device according to any one of claims 16 to 18, wherein the supporting device comprises at least one of a scanning table, a hospital bed, a wheelchair, or a bracket.

20. A computer-readable storage medium comprising a computer program stored thereon, wherein the computer program, when executed by a processor, causes the processor to implement the steps of the fragrance release method according to any one of claims 1 to 8.

21. A computer program product comprising a computer program, wherein the computer program, when executed by a processor, causes the processor to implement the steps of the fragrance release method according to any one of claims 1 to 8.
